Europäisches Patentamt
European Patent Office
Office européen des brevets

(19)

(11) Veröffentlichungsnummer: **0 052 836**
**A1**

(12)

# EUROPÄISCHE PATENTANMELDUNG

(21) Anmeldenummer: **81109640.3**

(22) Anmeldetag: **12.11.81**

(51) Int. Cl.³: **C 07 C 47/575**
C 07 C 49/86, C 07 C 79/36
C 07 C 87/50, C 07 C 121/75
C 07 C 149/32, C 07 C 149/34
C 07 C 149/415, C 07 D 213/65
C 07 D 277/68, C 07 D 317/64
//A01N53/00

(30) Priorität: **22.11.80 DE 3044009**

(43) Veröffentlichungstag der Anmeldung:
**02.06.82 Patentblatt 82/22**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt/Main 80(DE)**

(72) Erfinder: **Hüttelmaier, Thomas, Dr.**
**Kuckucksweg 19**
**D-6233 Kelkheim (Taunus)(DE)**

(72) Erfinder: **Mildenberger, Hilmar, Dr.**
**Fasanenstrasse 24**
**D-6233 Kelkheim (Taunus)(DE)**

(72) Erfinder: **Matterstock, Karl, Dr.**
**Lessingstrasse 38**
**D-6238 Hofheim am Taunus(DE)**

(54) **Neue Phenoxybenzaldehyde und Verfahren zu deren Herstellung.**

(57) Phenoxybenzaldehyde der Formel

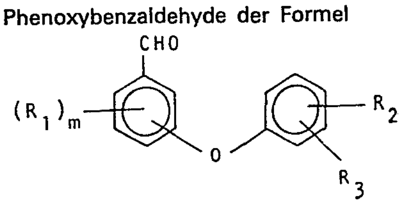

worin $R_1$ Halogen, CN, $NO_2$, (Cyclo)alkyl, Alkoxy, Alkylthio, Halogenalkyl oder Methylendioxy; $R_2$ Reste der Formeln

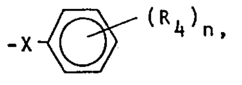

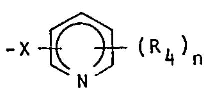 ,

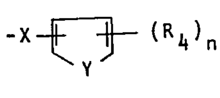

oder 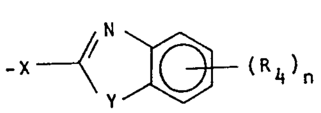 ,

$R_3$ H, Halogen, CN, $NO_2$, (Halogen)alkyl, Alkoxy, Alkylthio; $R_2$ + $R_3$ auch eine (un)gesättigte Kohlenwasserstoffkette; X −O−, −S−, −SO−, $-SO_2-$, $-OCH_2-$, $-CH_2O-$, $-CH_2-$, $-SCH_2-$, $-CH_2S-$, −CO−, −CH=CH−, −N− oder eine chemische Bindung; Y O oder S; $R_4$ Halogen, CN, $NO_2$, (Halogen)alkyl, (Halogen)-alkoxy, Cycloalkyl, Alkoxy, Alkylthio, Phenyl oder Methylendioxy, $R_5$ H, Alkyl oder Cycloalkyl, n 0-5 und m 0-4 bedeuten, sind Ausgangsstoffe für die Synthese wirksamer Insektizide und Akarizide.

EP 0 052 836 A1

# Neue Phenoxybenzaldehyde und Verfahren zu deren Herstellung

Die vorliegende Erfindung betrifft neue Phenoxybenzaldehyde der allgemeinen Formel (I)

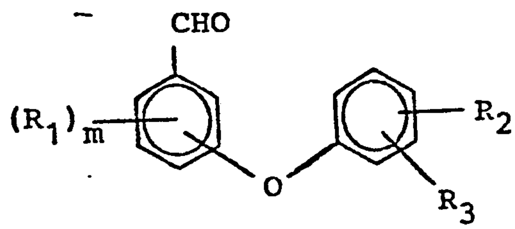

(I)

worin

$R_1$ Halogen, CN, $NO_2$, $(C_1-C_6)$-Alkyl, $(C_3-C_6)$-Cycloalkyl, $(C_1-C_6)$-Alkoxy, $(C_1-C_6)$-Alkylthio, Halogen $(C_1-C_2)$-alkyl oder Methylendioxy,

$R_2$ einen Rest der Formel

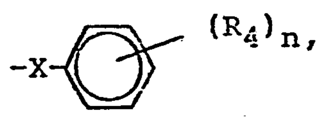

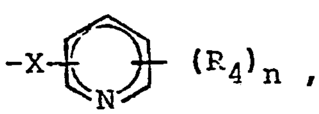

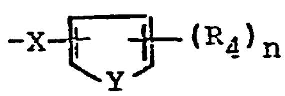

oder 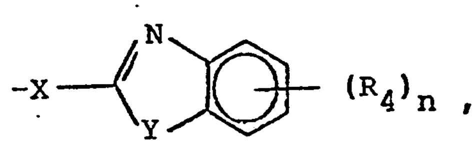

$R_3$ H, Halogen, CN, $NO_2$, $(C_1-C_6)$-Alkyl, $(C_1-C_6)$-Alkoxy, $(C_1-C_6)$-Alkylthio, Halogen $(C_1-C_2)$-alkyl oder zusammen mit $R_2$ eine ankondensierte ungesättigte oder gesättigte $C_4$-Kohlenwasserstoffkette, die anstelle einer -CH=Gruppe auch -N= enthalten kann,

X $-O-$, $-S-$, $-SO-$, $-SO_2-$, $-OCH_2-$, $-CH_2O-$, $-CH_2-$, $-SCH_2-$, $-CH_2S-$ $-CO-$, $-CH=CH-$, $-N(R_5)-$ oder eine einfache chemische Bindung,

Y -O- oder -S-,

$R_4$ Halogen, CN, $NO_2$, $(C_1-C_6)$-Alkyl, $(C_3-C_6)$-Cycloalkyl, Phenyl, $(C_1-C_6)$-Alkoxy, $(C_1-C_6)$-Alkylthio, Halogen-$(C_1-C_2)$-alkyl, Halogen$(C_1-C_2)$-alkoxy oder Methylendioxy

$R_5$ H,$(C_1-C_6)$-Alkyl oder $(C_1-C_6)$-Cycloalkyl,

n eine Zahl von 0 - 5 und

m eine Zahl von 0 - 4

bedeuten.

In den genannten Resten bedeutet "Halogen" vorzugsweise F, daneben auch Cl und Br. Halogen$(C_1-C_2)$alkylreste können bis zu 5 Halogenatome enthalten, vorzugsweise handelt es sich dabei um den $CF_3$-Rest. Falls m 0 ist, kommt als Rest R beispielsweise F, Cl, $CH_3$, $C_2H_5$, i-$C_3H_7$, $C_3$-, $C_5$- und $C_6$-Cycloalkyl, $OCH_3$, $OC_2H_5$, $SCH_3$, $SC_2H_5$ $CF_3$ und CHF-$CF_3$ in Frage. $R_3$ kann beispielsweise F, Cl, Br, $CH_3$, $C_2H_5$, i-$C_3H_7$, tert.$C_4H_9$, $OCH_3$, $OC_2H_5$, $SCH_3$, $SC_2H_5$ und $CF_3$ sein, bevorzugt jedoch ist $R_3$ = H. In $R_4$ bedeutet "Cycloalkyl" bevorzugt Cyclopropyl und "Halogenalkoxy" vorzugsweise $OCF_3$; $R_5$ steht bevorzugt für H, $CH_3$, $C_2H_5$ oder Cyclopropyl.

Gegenstand der Erfindung ist ferner ein Verfahren zur Herstellung der Phenoxybenzaldehyde der Formel (I), das dadurch gekennzeichnet ist, daß man gegebenenfalls substituierte Halogenbenzaldehyde der Formel

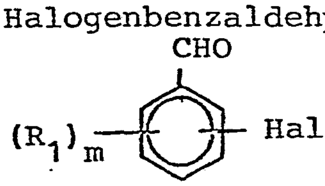

(II)

oder deren Acetale mit Phenolaten der Formel

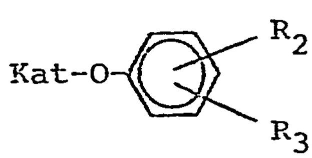

(III)

worin "Kat" ein Alkaliatom darstellt, gegebenenfalls in Anwesenheit eines Katalysators umsetzt.

In Formel II bedeutet Hal vorzugsweise Br, jedoch kommt auch Cl in Frage, "Kat" bedeutet vorzugsweise Natrium oder Kalium, wobei Natrium besonders bevorzugt ist. Das Phenolat III kann entweder als solches in die Reaktion eingesetzt oder "in situ" im Reaktionsgefäß hergestellt werden, beispielsweise indem man das entsprechende Phenol mit einem Alkalimetallhydroxid oder -alkoholat umsetzt und das gebildete Wasser bzw. den Alkohol gegebenenfalls azeotrop abdestilliert oder indem man es mit einem Alkalimetallhydrid reagieren läßt.

Die Reaktion von II und III kann in An- oder Abwesenheit eines aprotischen Lösungsmittels durchgeführt werden. Als solche kommen z.B. aromatische Kohlenwasserstoffe wie Toluol oder Xylol, heteroaromatische Stickstoffverbindungen wie Pyridin, 2,4,6-Trimethylpyridin, Chinoline oder Pyridin-N-oxid; Dialkylamide wie Dimethylformamid oder Dimethylacetamid; ferner N-Methylpyrrolidon, Dimethylsulfoxid oder Tetrahydrothiophen-1,1-dioxid oder Gemische aus diesen Lösungsmitteln in Betracht.

Als Katalysator eignen sich metallisches Kupfer sowie Kupfer(I)- und Kupfer(II)-verbindungen wie CuCl, CuBr, CuJ, $Cu_2O$, $CuF_2$, $CuCl_2$, $CuBr_2$, CuO oder Kupfer-p-chlorbenzoat.

Die Ausgangsstoffe II und III werden im wesentlichen in äquivalenten Mengen eingesetzt. Geringe Überschüsse einer der beiden Komponenten stören nicht, bringen aber auch keinen Vorteil.

Die Reaktionstemperatur kann innerhalb eines weiten Temperaturbereichs von Raumtemperatur bis ca. 250° variiert werden, liegt aber vorzugsweise zwischen 140° und 200°C. Die Reaktion ist je nach Umsetzungstemperatur nach 1 bis 24 Stunden beendet. Üblicherweise wird bei Normaldruck gearbeitet.

Die Ausgangsstoffe der Formel II und III (bzw. die letzteren zugrundeliegenden Phenole) sind bekannt bzw. werden nach bekannten Verfahren hergestellt (vgl. z.B. DT-OS 26 24 410, DT-PS 1 668 896 und DT-PS 21 36 828). Statt der Benzaldehyde II setzt man bevorzugt deren Acetale ein, die man in bekannter Weise durch Umsetzung mit niedermolekularen Alkoholen, wie z.B. Methanol, Ethanol, n-Propanol und Ethylenglykol erhält.

Die neuen Verbindungen fallen im allgemeinen in Form von hochviskosen Ölen an, die sich zum Teil nicht unzersetzt destillieren lassen. Sie können jedoch durch sogenanntes "Andestillieren", d.h. durch längeres Erhitzen unter vermindertem Druck auf mäßig erhöhte Temperaturen von den letzten Lösungsmittelresten befreit und auf diese Weise gereinigt werden. Gegebenenfalls werden die Rohprodukte durch Säulenchromatographie an Kieselgel gereinigt.

Die Phenoxybenzaldheyde der allgemeinen Formel (I) sind wertvolle Zwischenprodukte für Farbstoffe, Pflanzenschutzmittel, Kosmetika, Pharmazeutika im Human- und Veterinärbereich sowie für Wirkstoffe im Hygienesektor. Beispielsweise erhält man aus ihnen durch Reduktion substituierte Benzylalkohole, durch Umsetzung mit Grignard-Verbindungen α-Alkyl-, α-Alkenyl- bzw. α-Alkinylbenzylalkohole, durch Umsetzung mit HCN α-Cyano-benzylalkohole und durch Umsetzung mit Hydroxylamin Oxime, die ihrerseits z.B. mit Halogencyclopropancarbonsäuren zu pyrethrin-ähnlichen Verbindungen mit wertvollen insektiziden und akariziden Eigenschaften umgesetzt werden können.

Das erfindungsgemäße Verfahren wird durch folgende Beispiele näher erläutert:

Beispiel 1

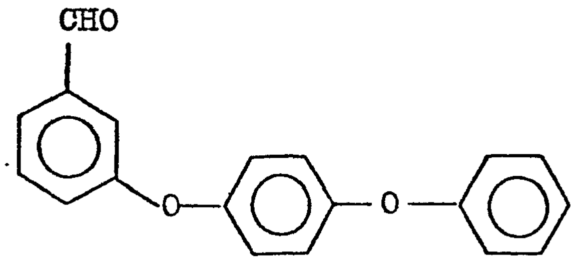

3-[4-Phenoxyphenoxy]benzaldehyd

Unter Schutzgasatmosphäre fügt man zu 15,0 g 80 % ige (0.5 Mol) Natriumhydridsuspension in 75 ml Dimethylformamid innerhalb von 45 min. eine Lösung aus 93.0 g (0.5 Mol) 4-Phenoxyphenol in 200 ml Dimethylformamid hinzu. Dann erhitzt man 15 min. auf 130° C und fügt bei dieser Temperatur 4,5 g (0.03 Mol) Kupfer [I]bromid und 39.5 g (0.5 Mol) Pyridin dazu. Danach werden innerhalb von 30 min. 114,5 g (0.5 Mol) 3-Brombenzaldehydacetal in 75 ml DMF zugetropft. Nach beendeter Zugabe erhitzt man 12 h auf Rückflußtemperatur. Nach dem Abkühlen zerstört man unter Eiskühlung das überschüssige Natriumhydrid mit Wasser, nimmt dann mit 300 ml Ether auf, wäscht 5 x 100 ml 2 n NaOH und dann mit 2 x 100 ml 2 n HCl. Anschließend wäscht man mit Wasser neutral, trocknet mit $Na_2SO_4$ und destilliert. Die Fraktion des Siedebereiches 165° - 222°C / 0.7-0.8 mbar ($n_D^{22}$ : 1.5979) nimmt man mit 250 ml Ether auf und schüttelt jeweils 5 min. mit 3 x 100 ml konz. HCl, wäscht neutral, trocknet mit $Na_2SO_4$ und zieht das Lösungsmittel ab. Man erhält 74.7 g (52%) eines gelben Oeles mit dem Brechungsindex $n_D^{20}$ : 1.6182

$^1$H-NMR ($CCl_4$) : $\delta$ = 9.85 (s; 1H), 7.60 - 6.70 (m; 13 H) ppm.-

IR ($CCl_4$) : 1700 $cm^{-1}$ (-CHO).-

| Analyse: $C_{19}H_{14}O_3$ (290) : | | ber.: | gef.: |
|---|---|---|---|
| | C : | 78,6 % | 78,3 % |
| | H : | 4,9 % | 5,1 % |

Beispiel 2

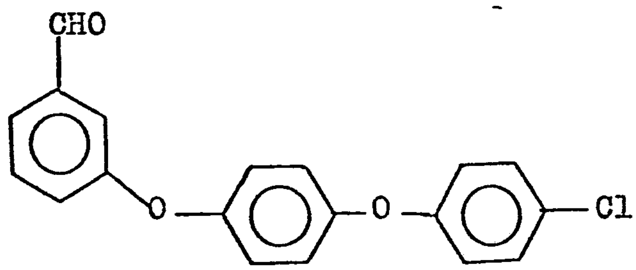

3-[4-(4-Chlorphenoxy)phenoxy]benzaldehyd

Unter Schutzgasatmosphäre fügt man zu 24.0 g 80 % ige (0.8 Mol) Natriumhydridsuspension in 100 ml Dimethylformamid innerhalb von 30 min. eine Lösung aus 176,4 g (0.8 Mol) 4-(4-Chlorphenoxy) phenol in 300 ml Dimethylformamid hinzu. Dann erhitzt man 15 min. auf 130°C und fügt bei dieser Temperatur 7,2 g (0.05 Mol) Kupfer -[I]bromid und 63.2 g (0.8 Mol) abs Pyridin dazu. Danach tropft man 148.0 g (0.65 Mol) 3-Brombenzaldehydacetal in 100 ml Dimethylformamid dazu und erhitzt 12 h unter Rückflußtemperatur. Danach kühlt man auf 0° ab und zerstört das überschüssige Hydrid mit Wasser. Man nimmt mit 800 ml

Ether auf und wäscht mit 5 x 100 ml 2 n NaOH und dann mit 2 x 100 ml 2 n HCl. Anschließend wäscht man mit Wasser neutral, trocknet mit $Na_2SO_4$ und zieht das Lösungsmittel ab. Vom Rohprodukt destilliert man die leichter siedenden Anteile bis zur Badtemperatur 210°C / 1.0 m bar ab. Den Rückstand nimmt man mit 300 ml Ether auf und schüttelt jeweils 5 min. mit 3 x 100 ml konz. HCl. Die Etherphase wäscht man neutral, trocknet mit $Na_2SO_4$ und zieht das Lösungsmittel ab. Man erhält 115.5 g (55 %) eines braunen Oeles. $^1$H-NMR ($CCl_4$) : $\delta$ = (s.1H), 7.60 - 6.70 (m; 12 H) ppm.- IR($CCl_4$) : 1700 (-CHO) $cm^{-1}$.-

Analyse $C_{19}H_{13}ClO_3$ (324.7)

| | ber.: | gef.: |
|---|---|---|
| C : | 70.3 % | 70.1 % |
| H : | 4.0 % | 4.1 % |
| Cl : | 10.9 % | 10.8 % |

Beispiel 3

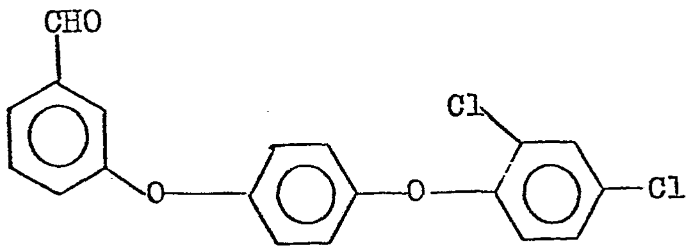

3-/4-(2,4-Dichlorphenoxy)phenoxy/benzaldehyd

Unter Schutzgasatmosphäre fügt man zu 18.0 g 80 %iger (0.6 Mol) Natriumhydridsuspension in 75 ml Dimethylformamid innerhalb von 30 min. eine Lösung aus 153.0 g (0.6 Mol) 4-(2,4-Dichlorphenoxy)phenol in 225 ml Dimethylformamid hinzu. Dann erhitzt man 15 min. auf 130°C

und fügt bei dieser Temperatur 5.4 g (0.038 Mol) Kupfer-[I]bromid und 47,4 g (0.6 Mol) Pyridin und innerhalb von 30 min. 111.0 g (0.6 Mol) 3-Brombenzaldehydacetal in 75 ml Dimethylformamid dazu und erhitzt 12 h unter Rückfluß. Nach dem Abkühlen auf 0° zerstört man das überschüssige Hydrid mit Wasser setzt 600 ml Ether zu und extrahiert mit 2 x 200 ml 2 n NaOH. Danach wäscht man mit Wasser neutral, trocknet über $Na_2SO_4$ und zieht das Lösungsmittel ab. Vom Rohprodukt destilliert man die leichtersiedenden Anteile bis zur Badtemperatur 220°C/1.0 mbar ab. Den Rückstand nimmt man mit 600 ml Ether auf und schüttelt mit 2 x 300 ml konz. HCl, jeweils 5 min. aus. Die Etherphase wäscht man mit Wasser neutral, trocknet über $Na_2SO_4$ und zieht das Lösungsmittel ab.

Man erhält 154.0 g (71 %) eines dunkelbraunen Oeles.

$^1$H-NMR ($CCl_4$) : $\delta$ = 9.80 (s; 1 H), 7.60 - 6.70 (m; 11 H) ppm .

IR ($CCl_4$) : 1720 (-CHO) $cm^{-1}$.-

<u>Analyse</u> $C_{19}H_{12}Cl_2O_3$ (359)

| | ber.: | gef.: |
|---|---|---|
| C : | 63,5 % | 63.8 % |
| H : | 3,4 % | 3,4 % |

<u>Beispiel 4</u>

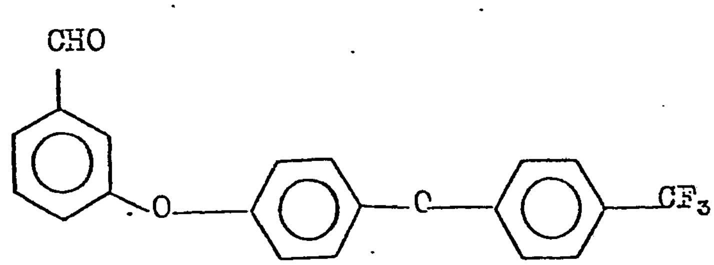

3-[4-(4-Trifluormethyl(phenoxy)phenoxy]benzaldehyd

Unter Schutzatmosphäre fügt man zu 6.0 g 80 % iger (0.2 Mol) Natriumhydridsuspension in 25 ml 1-Methylpyrrolidon innerhalb von 25 min. eine Lösung aus 50.8 g (0.2 Mol) 4-(4-Trifluormethylphenoxy ) phenol in 75 ml 1-Methylpyrrolidon hinzu. Anschließend erhitzt man 15 min. auf 150°C und fügt dann 1.8 g (0.0125 Mol) Kupfer [I]-bromid und 15.8 g (0.2 Mol) Pyridin dazu. Danach tropft man innerhalb von 30 min. 55.8 g (0.2 Mol) 3-Brombenzaldehydacetal in 25 ml 1-Methylpyrrolidin dazu und erhitzt 12 h auf 180°C. Nach dem Abkühlen saugt man die Feststoffe ab und destilliert von Filtrat die leichter siedenden Bestandteile bis zur Badtemperatur 220°C / 0.2 mbar ab. Den Rückstand nimmt man mit 200 ml Ether auf, schüttelt mit 3 x 50 ml konz. HCl jeweils 5 min aus, wäscht die Etherphase mit Wasser neutral, trocknet über $Na_2SO_4$, zieht das Lösungsmittel ab und erhält 44.9 g (63 %) eines braunschwarzen zähen Oeles.

$^1$H-NMR ($CCl_4$) : $\delta$ = 9.9 (s; 1 H), 7.70 - 6.80 (m; 12 H) ppm.-

IR ($CCl_4$) : 1720 (-CHO) $cm^{-1}$.-

Beispiel 5

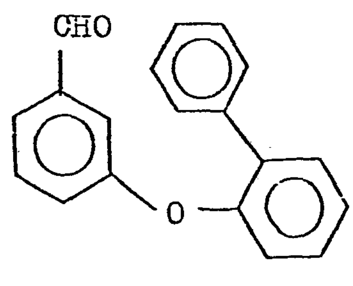

3-[2-Phenylphenoxy]-benzaldehyd

Unter Schutzatmosphäre fügt man zu 18.0 g 80 %iger (0.6 Mol) Natriumhydridsuspension in 100 ml N-Methylpyrrolidon innerhalb von 25 min. eine Lösung aus 102.0 g (0.6 Mol) 2-Hydroxybiphenyl und 100 ml N-Methylpyrrolidon. Danach erhitzt man 15 min. auf 150°C und fügt dann 5.4 g (0.036 Mol) Kupfer [I]-bromid und 47.4 g (0.6 Mol) abs. Pyridin dazu, schließlich tropft man innerhalb von 30 min. 137.4 g (0.6 Mol) 3-Brombenzaldehydacetal in 100 ml N-Methylpyrrolidon dazu und erhitzt 12 h auf 180°C. Von der erhaltenen Reaktionsmischung saugt man die Feststoffe ab und destilliert die leichter siedenden Komponenten bis zur Badtemperatur von 210°C / 0.2 mbar ab. Den Rückstand nimmt man mit 200 ml Ether auf und schüttelt jeweils 5 min. mit 3 x 50 ml konz. HCl aus. Die Etherphase wäscht man danach mit 3 x 100 ml 2 n NaOH und dann mit Wasser neutral, trocknet und zieht dann das Lösungsmittel ab. Man erhält 40.5 g (25 %) eines braunen Oeles.

$^1$H-NMR : ($CCl_4$) : $\delta$ = 9.70 (s ; 1 H), 7.60 - 6.70 (m, 13 H).-

IR ($CCl_4$) : 1710 (-CHO) $cm^{-1}$.-

Beispiel 6

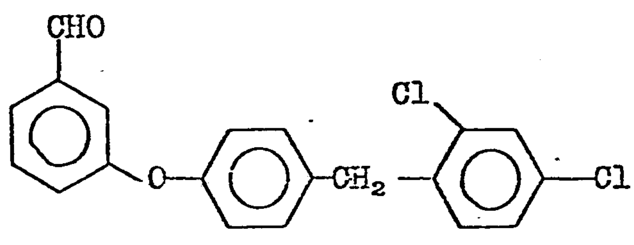

3-[4-(2,4-Dichlorbenzyl)phenoxy]benzaldehyd

Unter Schutzatmosphäre fügt man zu 6.6 g 80 %iger

(0.22 Mol) Natriumhydridsuspension in 50 ml N-Methylpyrrolidon innerhalb von 45 min. 50.6 g (0.2 Mol) 4-(2',4'-Dichlorbenzyl)phenol in 50 ml N-Methylpyrrolidon hinzu und erhitzt 15 min. auf 130°C Dann fügt man 1.8 g (0.012 Mol) Kupfer /I/-bromid und 15.8 g (0.2 Mol) abs. Pyridin und schließlich innerhalb von 15 min. 45.8 g (0.2 Mol) 3-Brombenzaldehydacetal in 25 ml N-Methylpyrrolidon hinzu. Man erhitzt 12 h auf 180°C, kühlt danach ab, saugt die Festprodukte ab und destilliert vom Filtrat die niedriger siedenden Komponenten bis zur Badtemperatur 200°C / 0.2 mbar ab. Den Rückstand nimmt man mit 250 ml Ether auf und wäscht mit 2 n NaOH Phenolfrei, dann schüttelt man mit 2 x 50 ml konz. HCl, wäscht die Etherphase neutral, trocknet über $Na_2SO_4$ und zieht das Lösungsmittel ab. Man erhält 7.2 g (11 %) eines gelben zähen Oeles.

$^1$H-NMR ($CDCl_3$) : $\delta$ = 9.80 (s; 1 H), 7.60 - 6.40 (m, 11 H), 3.90 (s, 2 H).-

IR ($CDCl_3$) : 1720 (-CHO) $cm^{-1}$.-

Beispiel 7

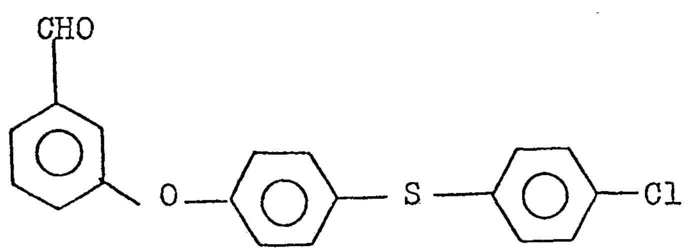

3-/4-(4-Chlorthiophenol)phenoxy/benzaldehyd

Unter Schutzgasatmosphäre fügt man zu 9.5 g (0.3 Mol; 80 %iger) Natriumhydridsuspension und 65 ml N-Methylpyrrolidon eine Lösung aus 74.0 g (0.3 Mol) 4'-Chlor-4-phenylthiophenol in 80 ml N-Methylpyrrolidon hinzu und erhitzt 15 min. auf 150°C. Dann fügt man 3.1 g (0.02 Mol) Kupfer /I/ bromid und 24 ml (0.3 Mol) Pyridin hinzu. Schließlich tropfte man eine Lösung aus 73.0 g (0.3 Mol) in 80 ml N-Methylpyrrolidon dazu und erhitzt 9.5 h auf 180°C. Danach kühlt man ab, saugt die Festprodukte ab. Vom Filtrat destilliert man die niedrig siedenden Bestandteile bis zur Badtemperatur 220°C / 0.1 mbar ab. Den Rückstand nimmt man mit 300 ml Methylenchlorid auf und wäscht mit 2n NaOH phenolfrei. Danach schüttelt man mit 2 x 100 ml konz. HCl aus, wäscht die Methylenchloridphase neutral trocknet über $Na_2SO_4$ und zieht das Lösungsmittel ab. Man erhält 82.7 g (81 %) eines braunen Oeles.

$^1$H-NMR: ($CDCl_3$) : = 9.90 (s; 1 H); 8.00 - 6.90 (m; 12 H).-

IR ($CHCl_3$) : 1720 (-CHO) $cm^{-1}$.-

Beispiel 8

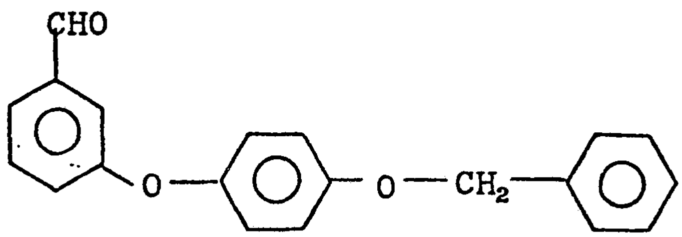

3-[4-Benzyloxyphenoxy]benzaldehyd

Unter Schutzgasatmosphäre fügt man zu 7.5 g 80 %iger (0.25 Mol) Natriumhydridsuspension in 30 ml Dimethylformamid innerhalb von 30 min. eine Lösung aus 50.0 g (0.25 Mol) 4-Benzyloxyphenol in 75 ml Dimethylformamid hinzu. Dann erhitzt man 15 min. auf 130°C und fügt dann bei dieser Temperatur 1.8 g (0.0125 Mol) Kupfer [I] bromid und 19.8 g (0.25 Mol) Pyridin und innerhalb von 20 min. 57.3 g (0.25 Mol) 3-Brombenzaldehydacetal in 100 ml Dimethylformamid hinzu. Man erhitzt 12 h auf Rückflußtemperatur, kühlt dann auf 0° ab, zerstört das überschüssige Natriumhydrid vorsichtig mit Wasser, setzt 300 ml Ether zu und extrahiert mit 2 x 100 ml 2 n NaOH. Danach wäscht man mit $H_2O$ neutral, trocknet über $Na_2SO_4$ und zieht das Lösungsmittel ab. Vom Rohprodukt zieht man die leichtersiedenden Bestandteile bis zur Badtemperatur 210°C/ 1.0 m bar ab. Den Rückstand nimmt man mit 300 ml Ether auf und schüttelt mit 3 x 100 ml konz. HCl jeweils 5 min. aus. Die Etherphase wäscht man neutral, trocknet über $Na_2SO_4$ und zieht das Lösungsmittel ab. Man erhält 40.3 g (53 %) eines gelbbraunen zähen Oeles:

$^1$H-NMR ($CCl_4$) : $\delta$ = 9.80 (s; 1 H); 7.60 - 6.70 (m; 13 H)
4.90 (s; 2 H) ppm.-

IR ($CHCl_3$) : 1710 (-CHO) $cm^{-1}$.-

<u>Analyse:</u> $C_{20}H_{16}O_3$ (304) :

| | ber.: | gef.: |
|---|---|---|
| C : | 78.9 % | 79.2 % |
| H : | 5.3 % | 5.5 % |

<u>Beispiel 9</u>

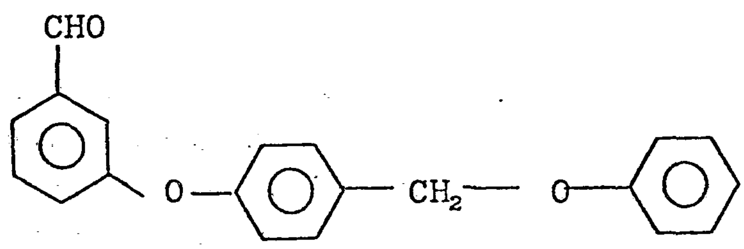

3-(4-Phenoxybenzyloxy) benzaldehyd

Unter Schutzgasatmosphäre fügt man zu 7.5 g 80 %iger Natriumhydridsuspension (0.25 Mol) in 30 ml Dimethylformamid innerhalb von 30 min. eine Lösung aus 50.0 g (0.25 Mol) 4-Hydroxybenzylphenylether in 75 ml Dimethylformamid hinzu. Dann erhitzt man 15 min. auf 130°C und fügt dann bei dieser Temperatur 1.8 g (0.0125 Mol) Kupfer /I/-bromid und 19.8 g (0.25 Mol) Pyridin und innerhalb von 20 min. 57.3 g (0.25 Mol) 3-Brombenzaldehydacetal in 100 ml Dimethylformamid hinzu. Man erhitzt 18 h auf Rückflußtemperatur, kühlt dann auf 0°C ab, zerstört das überschüssige Natriumhydrid vorsichtig mit Wasser, setzt 300 ml Ether hinzu und extrahiert mit 2 x 100 ml 2 n NaOH. Danach wäscht man neutral, trocknet über $Na_2SO_4$, zieht

das Lösungsmittel ab und destilliert vom Rohprodukt die niedrigsiedenden Fraktionen bis zur Badtemperatur 210°C / 1.0 mbar ab. Den Rückstand nimmt man mit 300 ml Ether auf und schüttelt mit 3 x 100 ml konz. HCl jeweils 5 min. aus. Die Etherphase wäscht man neutral, trocknet über $Na_2SO_4$ und zieht das Lösungsmittel ab. Man erhält 4.5 g (60 %) eines hochviskosen helbbraunen Oeles.

$^1$H-NMR ($CCl_4$) : $\delta$ = 9.80 (s; 1 H); 7.60 - 6.70 (m; 13 H); 4.95 (s; 2 H); ppm.-

IR ($CCl_4$) : 1715 (-CHO) $cm^{-1}$.-

<u>Analyse:</u> $C_{20}H_{16}O_3$ (304) :

| | ber.: | gef.: |
|---|---|---|
| C : | 78.9 % | 81.2 % |
| H : | 5.3 % | 5.1 % |

<u>Beispiel 10</u>

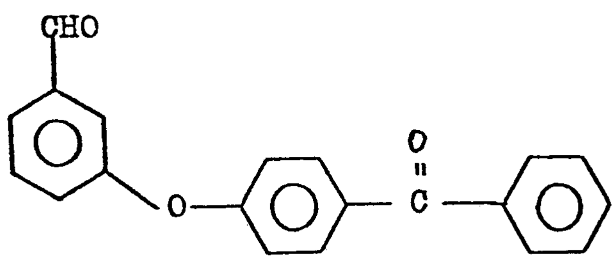

3-(4-Benzophenonoxy) benzaldehyd

Unter Schutzgasatmosphäre fügt man zu 7.5 g 80 %iger (0.25 Mol) Natriumhydridsuspension in 30 ml Dimethylformamid innerhalb von 30 min. eine Lösung aus 49.5 g (0.25 Mol) 4-Hydroxybenzophenon in 75 ml Dimethylformamid und erhitzt 16 min. auf 130°C. Dann fügt man 1.8 g (0.0125 Mol) Kupfer /I_7bromid, 19.8 g (0.25 Mol) Pyridin und schließlich 57.3 g (0.25 Mol) 3-Brombenzaldehydacetal in 50 ml Dimethylformamid innerhalb von 20 min. hinzu. Man erhitzt 12 h auf Rückfluß, kühlt dann auf 0°C ab und hydrolysiert vorsichtig das überschüssige Natriumhydrid. Dann setzt man 300 ml Ether zu, wäscht mit 2 x 100 ml 2 n NaOH und anschließend mit Wasser neutral. Nach dem Trocknen über $Na_2SO_4$ zieht man den Ether ab und destilliert die leichtsiedenden Fraktionen bis zur Badtemperatur 210°C / 1.0 mbar ab. Den Rückstand nimmt man mit 300 ml Ether auf und schüttelt mit 3 x 100 ml konz. HCl jeweils 5 min. aus. Die Etherphase wäscht man neutral, trocknet über $Na_2SO_4$ und zieht das Lösungsmittel ab. Man erhält 60.0 g (79 %) eines zähen braunen Oeles.

$^1$H-NMR ($CCl_4$) : $\delta$ = 9.80 (s; 1 H); 7.70 - 6.70 (m; 13 H) ppm.-

IR ($CCl_4$) : 1720 (-CHO) $cm^{-1}$.-

Analyse: $C_{20}H_{14}O_3$ (302) :

| | ber.: | gef.: |
|---|---|---|
| C: | 79.5 % | 79.8 % |
| H: | 4.6 % | 4.7 % |

Beispiel 11

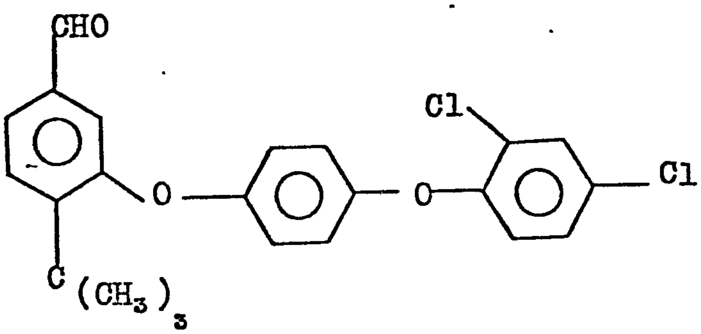

3-/4-(2,4-Dichlorphenoxy)phenoxy7-4-tert.-butyl-benzaldehyd

Unter Schutzgasatmosphäre fügt man zu 3.9 g 80 %iger (0.13 Mol) Natriumhydridsuspension in 25 ml N-Methylpyrrolidon innerhalb von 30 min. eine Lösung aus 33.2 g (0.13 Mol) 4-(2,4-Dichlorphenoxy)phenol in 50 ml N-Methylpyrrolidon hinzu und erhitzt 15 min. auf 130°C. Bei dieser Temperatur fügt man 0.9 g (0.00625 Mol) Kupfer-/I_7bromid und 9.5 g (0.12 Mol) Pyridin und innerhalb von 15 min. 34.2 g (0.12 Mol) 3-Brom-4-tert.butyl-benzaldehyd-acetal in 25 ml N-Methylpyrrolidon hinzu. Anschließend erhitzt man 12 h auf 180°C. Nach dem Abkühlen saugt man die Feststoffe ab und destilliert vom Filtrat die leichtsiedenden Bestandteile bis zur Badtemperatur 210°C / 1.0 mbar ab. Den Rückstand nimmt man mit 250 ml Ether auf schüttelt jeweils 5 min. mit 3 x 50 ml konz. HCl aus. Die Etherphase wäscht man neutral, trocknet über $Na_2SO_4$ zieht das Lösungsmittel ab und gewinnt 38.9 g (78 %) eines zähen braunen Oeles.

$^1$H-NMR ($CDCl_3$) : $\delta$ = 9.85 (s; 1 H), 7.60 - 6.70 (m; 10 H), 1.45 (S; 9 H) ppm.-

IR ($CCl_4$) : 1700 (-CHO) $cm^{-1}$

Analyse: $C_{23}H_{20}Cl_2O_3$ (415)

| | ber.: | gef.: |
|---|---|---|
| C: | 66.5 % | 66.9 % |
| H: | 4.8 % | 4.6 % |
| Cl: | 17.1 % | 17.3 % |

Beispiel 12

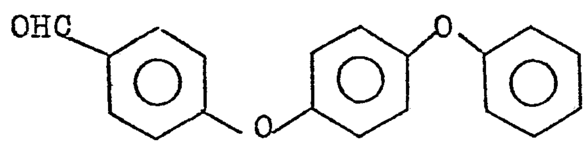

4-/4-(4-Phenoxy)phenoxy7benzaldehyd

Unter Schutzgasatmosphäre fügt man zu 7.5 g 80 %iger (0.25 Mol) Natriumhydridsuspension in 75 ml Dimethylformamid innerhalb von 20 min. eine Lösung aus 46.5 g (0.25 Mol) 4-Phenoxyphenol in 100 ml Dimethylformamid hinzu. Dann erhitzt man 15 min. auf 130°C und fügt bei dieser Temperatur 2.25 g (0.015 Mol) Kupfer /I_7bromid und 19.8 g (0.25 Mol) Pyridin dazu. Danach werden innerhalb von 80 min. 57,3 g (0.25 Mol) 4-Brombenzaldehydacetal in 50 ml Dimethylformamid zugetropft. Nach beendeter Zugabe erhitzt man 12 h auf Rückflußtemperatur. Nach dem Abkühlen zerstört man unter Eiskühlung das überschüssige Natriumhydrid mit Wasser, nimmt dann mit 200 ml Ether auf, wäscht 5 x 50 ml 2 n NaOH und dann mit

2 x 100 ml 2 n HCl und anschließend mit Wasser neutral. Nach dem Trocknen über $Na_2SO_4$ zieht man das Lösungsmittel ab und destilliert die leichter flüchtigen Komponenten bis zur Badtemperatur 210°C / 1.0 mbar ab. Den Rückstand nimmt man mit 200 ml Ether auf, schüttelt jeweils 5 min. mit 3 x 75 ml konz. HCl aus, wäscht neutral, trocknet über $Na_2SO_4$ und erhält ein gelbes zähes Oel.

$^1H$-NMR ($CCl_4$) : $\delta$ = 9.85 (s; 1 H), 7.60 - 6.70 (m; 13 H) ppm.-

IR ($CCl_4$) : 1705 (-CHO) $cm^{-1}$.-

Analyse: $C_{19}H_{14}O_3$ (290)

| | ber.: | gef.: |
|---|---|---|
| C: | 78,6 % | 78,9 % |
| H: | 4,9 % | 5,0 % |

In analoger Weise können unter Verwendung entsprechender Ausgangsstoffe folgende Verbindungen erhalten werden:

| Beispiel | $(R_1)_m$ | $R_2$ | $R_3$ | Phys. Daten |
|---|---|---|---|---|
| 13 | H | 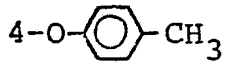 | H | 1H-NMR ($CCl_4$): $\delta$ = 9.80 (s; 1H), 7.50-6.60 (m;12H) 2.25 (s; 3H) ppm. |
| 14 | H | 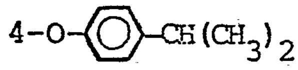 | H | 1H-NMR ($CCl_4$): $\delta$ = 9.90 (s; 1H) 7.70-6.60 (m; 12H), 3.30 (m; 1H), 1.30 (d; 6H) ppm.- |
| 15 | H | 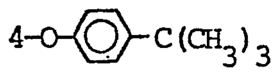 | H | |
| 16 | H | 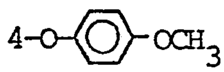 | H | 1H-NMR ($CCl_4$): $\delta$ =9.90 (s; 1H), 7.60-6.60 (m; 12H), 3.75 (s; 3H) ppm.- |
| 17 | H | 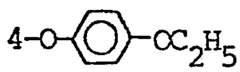 | H | |
| 18 | H | 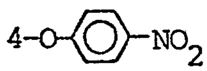 | H | |
| 19 | H | 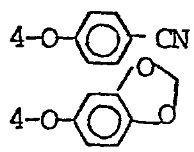 | H | |
| 20 | H | | H | |
| 21 | H | 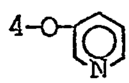 | H | 1H-NMR ($CCl_4$): $\delta$= 9.90 (s; 1H), 8.10 (m; 2H), 7.60-6.60 (m; 10H) ppm.- |
| 22 | H | 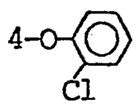 | H | 1H-NMR ($CCl_4$): $\delta$=9.85 (s; 1H), 7.60-6.80 (m; 12H) ppm.- |
| 23 | H | 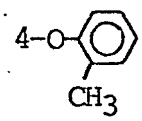 | H | 1H-NMR ($CCl_4$): $\delta$=9.85 (s; 1H), 7.60-6.70 (m; 12H) 2.20 (s; 3H) ppm.- |
| 24 | H | 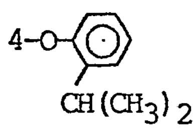 | H | 1H-NMR ($CCl_4$): $\delta$=9.90 (s; 1H), 7.60-6.70 (m; 12H) 3.30 (m; 1H), 1.30 (d; 6H) ppm.- |
| 25 | H | 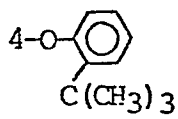 | H | |
| 26 | H | 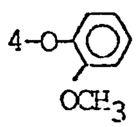 | H | 1H-NMR ($CCl_4$): $\delta$= 9.90 (s; 1H), 7.50-6.60 (m; 12H) 3.70 (s; 3H) ppm |

| Beisp. | $R_1)_m$ | $R_2$ | $R_3$ | Phys. Daten |
|---|---|---|---|---|
| 27 | H | 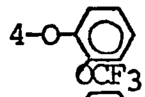 | H | |
| 28 | H | 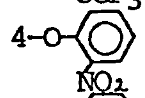 | H | |
| 29 | H | 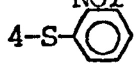 | H | |
| 30 | H | 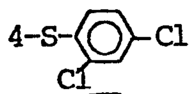 | H | |
| 31 | H | 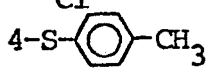 | H | |
| 32 | H | 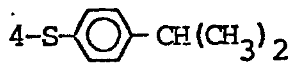 | H | |
| 33 | H | 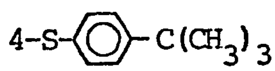 | H | |
| 34 | H | 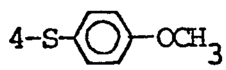 | H | |
| 35 | H | 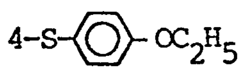 | H | |
| 36 | H | 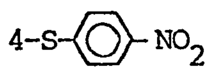 | H | |
| 37 | H | 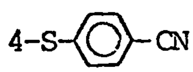 | H | |
| 38 | H | 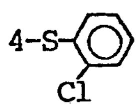 | H | |
| 39 | H | 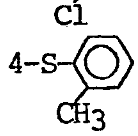 | H | |
| 40 | H | 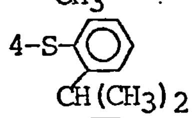 | H | |
| 41 | H | 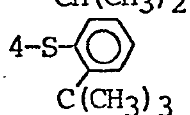 | H | |
| 42 | H | 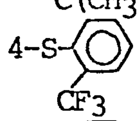 | H | |
| 43 | H | 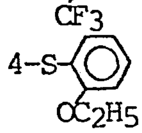 | H | |
| 44 | H | 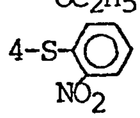 | H | |
| 45 | H | 3,4-CH=CH-CH=CH- | | 1H-NMR ($CCl_4$): $\delta$ =9.90 (s; 1H), 7.90-6.90 (m; 11H) ppm.- |

| Beisp. | $(R_1)_m$ | $R_2$ | $R_3$ | Phys. Daten |
|---|---|---|---|---|
| 46 | H | 2,3-CH=CH-CH=CH- | | 1H-NMR ($C Cl_4$): =9.80 (s;1H) 8.10-6.70 (m;11H) ppm.- |
| 47 | H | 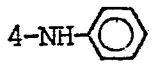 | H | |
| 48 | H | 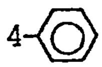 | H | 1H-NMR ($C Cl_4$): =9.90 (s;1H) 7.90-6.70 (m;12H) ppm.- |
| 49 | H | 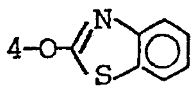 | H | 1H-NMR ($CDCl_3$): =9.90 (s;1H) 8.20-7.00 (m;12H) ppm.- |
| 50 | 4-F | 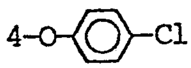 | H | 1H-NMR ($C Cl_4$): =9.90 (s;1H), 8.20-670 (m; 11H) ppm.- |
| 51 | 4-F | 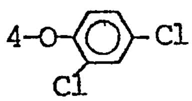 | H | 1H-NMR ($C Cl_4$): =9.80 (s;1H) 8.20-6.60 (m;10H) ppm.- |
| 52 | H | 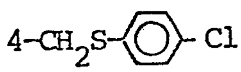 | H | |
| 53 | H | 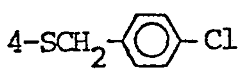 | H | |
| 54 | H | 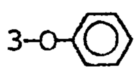 | H | |
| 55 | H | 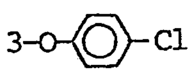 | H | |
| 56 | H | 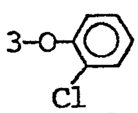 | H | |
| 57 | H | 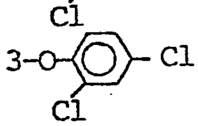 | H | |
| 58 | F | 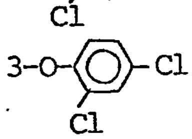 | H | |

Patentansprüche:

1. Phenoxybenzaldehyde der allgemeinen Formel (I)

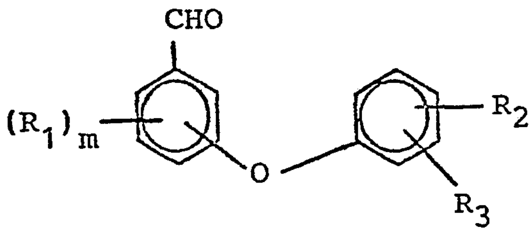

(I)

worin

$R_1$ Halogen, CN, $NO_2$, $(C_1-C_6)$-Alkyl, $(C_3-C_6)$-Cycloalkyl, $(C_1-C_6)$-Alkoxy, $(C_1-C_6)$-Alkylthio, Halogen $(C_1-C_6)$-alkyl oder Methylendioxy,

$R_2$ einen Rest der Formel

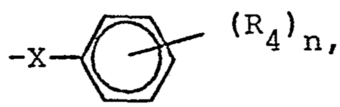

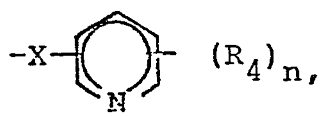

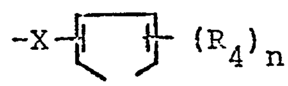

oder 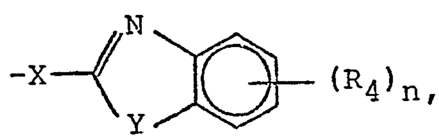

$R_3$ H, Halogen, CN, $NO_2$, $(C_1-C_6)$-Alkyl, $(C_1-C_6)$-Alkoxy, $(C_1-C_6)$-Alkylthio, Halogen $(C_1-C_2)$-alkyl oder zusammen mit $R_2$ eine ankondensierte ungesättigte oder gesättigte $C_4$-Kohlenwasserstoffkette, die anstelle einer -CH=Gruppe auch -N= enthalten kann,

X -O-,-S-,-SO-,$-SO_2-$,$-OCH_2-$,$-CH_2O-$,$-CH_2-$,$-SCH_2-$,$-CH_2S-$ -CO-,-CH=CH-, $-N(R_5)-$ oder eine einfache chemische Bindung,

Y -O- oder -S-,

$R_4$ Halogen, CN, $NO_2$, $(C_1-C_6)$-Alkyl, $(C_3-C_6)$-Cycloalkyl, Phenyl, $(C_1-C_6)$-Alkoxy, $(C_1-C_6)$-Alkylthiom Halogen-$(C_1-C_2)$-alkyl, Halogen$(C_1-C_2)$-alkoxy oder Methylendioxy

$R_5$ H, $(C_1-C_6)$-Alkyl oder $(C_1-C_6)$-Cycloalkyl,
n eine Zahl von 0 - 5 und
m eine Zahl von 0 - 4
bedeuten.

2. Verfahren zur Herstellung von Phenoxybenzaldehyden der Formel I, dadurch gekennzeichnet, daß man gegebenenfalls substituierte Halogenbenzaldehyde der Formel

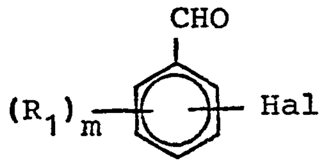

(II)

oder deren Acetale mit Phenolaten der Formel

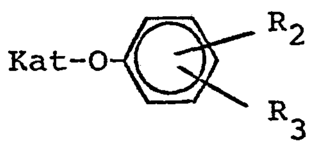

(III)

worin "Kat" ein Alkaliatom darstellt, gegebenenfalls in Anwesenheit eines Katalysators umsetzt.

3. Verwendung von Verbindungen der Formel I als Ausgangsstoffe für die Herstellung von Insektiziden und Akariziden.

0052836

Europäisches Patentamt

# EUROPÄISCHER RECHERCHENBERICHT

Nummer der Anmeldung

EP 81 10 9640

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | CH - A - 205 415 (GEIGY) | |
| | * Ganzes Dokument * | 1,2 |
| | -- | |
| X | FR - A - 818 032 (GEIGY) | |
| | * Seite 2, Spalte 1, Zeilen 16-52, Seite 2, Spalte 2, Zeilen 53,54, 64-66 * | 1,2 |
| | -- | |
| X | US - A - 2 894 977 (W. SIEDEL) | |
| | * Spalte 12, Zeilen 45-70 * | 1 |
| | -- | |
| A | DE - A - 2 620 791 (CIBA-GEIGY) | |
| | * Ansprüche 1,4; Seite 6, Zeilen 20-22 * | 1-3 |
| | ---- | |

KLASSIFIKATION DER ANMELDUNG (Int. Cl.³)

C 07 C 47/575
49/86
79/36
87/50
121/75
149/32
149/34
149/415
C 07 D 213/65
277/68
317/64
C 07 C 45/70
76/02
120/00
148/00//
A 01 N 53/00

RECHERCHIERTE SACHGEBIETE (Int Cl.³)

C 07 C 47/00
C 07 C 149/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 17-02-1982 | WRIGHT |